# EUROPEAN PATENT APPLICATION

(11) **EP 4 714 409 A1**
(43) Date of publication of application: **25.03.2026**
(21) Application number: 25183649.0
(22) Date of filing: 18.06.2025
(51) Int. Cl.: A61F 13/15

(54) **AIRLAID PRODUCED IN LINE**

(30) Priority: 18.09.2024 IT 202400020848
(71) Applicant: GDM S.P.A., 40133 Bologna (IT)
(72) Inventor: ZAVALLONI, Alessandro, 40133 Bologna (IT)
(74) Representative: Emmi, Mario

(57) **Abstract**

The present invention concerns a production line 100 (also called converter 100) for manufacturing absorbent products for sanitary use wherein the absorbent core is produced directly in special processing sections of said converter 100.

## Description

### Scope of the invention

The present invention concerns the technical field inherent in the production of absorbent products for sanitary use, preferably products with high absorbency, in particular absorbent products for feminine hygiene.

In particular, the invention refers to an innovative production method and production line for manufacturing absorbent products for sanitary use, in particular for female use, in which the waste of production material is greatly reduced while having highly flexible production plants, for example for format changes.

### A brief outline of the prior art

The use of the so-called "airlaid" material in the production of various products has long been known, including the absorbent products for sanitary use. This material is in fact used to create the pad, therefore the absorbent core, of the final absorbent product for sanitary use.

The "airlaid" material is a well-known material free of plastics and composed of cellulose fibres that are treated with air. SAP material can be integrated if necessary.

This airlaid material is therefore characterised by being in the form of a layer of very uniform fibres, which is compact and produced with plants developed specifically to deposit these uniform layers of fibres and any SAP.

At the current state of the art, in the production of absorbent products for sanitary use, mother reels of airlaid tape are generally used and with the entire tape suitable for constituting the absorbent pad or core simply by shaping it to size. The airlaid mother reel is therefore loaded into the machine and unrolled so that it is cut to size for the formation of the pad (herein referred to as the absorbent core). The pad, cut to size, is inserted between two layers of material in order to obtain the single finished absorbent products for sanitary use ready to be packaged and marketed.

In particular, at the current state of the art, the "airlaid" production lines are external to the converter (i.e. the production line) that produces the final absorbent product for sanitary use.

These "airlaid" reels, which will then constitute, as mentioned, the absorbent core of the finished product, are therefore produced on specific lines and with these reels that are often very wide (e.g. 2-3 m). Said reels are purchased in various formats and subsequently slid, and on order based on the necessary widths, in the converter. This results in low production flexibility and high material waste.

In particular, changing the width, grammage, presence or absence and quantity of SAP requires those who produce the "raw material" to vary these specifications upstream and to have in stock all the purchased material formats (reels) which in themselves already make up a continuous tape of airlaid absorbent cores possibly provided with SAP.

There is therefore a high cost of production, a high cost of logistics and economic immobilisation, as well as a very low flexibility in changing recipes.

Another extremely important problem is that since the machines are fed with reels formed by airlaid tape, but since the finished products are all shaped, a high quantity of non-recoverable "noble" material waste is necessarily generated. In other words, the starting tape is a noble material suitable for forming the core of the absorbent product but, as mentioned, the step of shaping the tape for making the final shape and size of the pad implies a high waste of noble material, given that the shape of the pad to be inserted into the final absorbent product must be obtained from the tape. It is therefore difficult to find tapes whose width minimizes waste.

### Summary of the invention

It is therefore the object of the present invention to provide a production line (also called converter line or simply converter) and relative method that allows to solve, at least in part, the aforementioned technical drawbacks.

In particular, it is the object of the present invention to provide a production line and relative production method of absorbent products for sanitary use, for example pads for female use and/or diapers in general, capable of integrating a flexible production system of the absorbent core in said production line such that, moreover, the waste of material is minimal.

Another object of the present invention is to provide a production line and relative production method in which there is consequently a high flexibility and therefore there is a high possibility of changing "recipes" (therefore, format change, for example).

A further object of the present invention is to provide a production line and relative production method in which it is possible to use SAP of any grammage, thus even particularly fine SAP, without this implying production problems.

These and other purposes are therefore achieved with the method and the production line in accordance with the independent claims.

In particular, the object of the present invention is a method for manufacturing, in a converter line (1, 2, 3, 4), an absorbent product for sanitary use, for example pads for female use, the method comprising the steps of:

Directly producing in said converter line at least one continuous tape (200) comprising a plurality of absorbent cores (201) arranged in succession according to a certain pitch;

Cutting out from said tape (200) the absorbent cores (201) in such a way as to obtain single absorbent cores (201') separated from the tape;

Sending said single absorbent cores (201') towards a subsequent manufacturing section of the final absorbent product for sanitary use.

In this way, all the aforementioned technical inconveniences are solved.

In particular, the absorbent core is now produced directly in line in the converter.

In this way, it is no longer necessary to have to use airlaid mother reels already made upstream and stored in the warehouse, since the same converter line can now produce an absorbent core 201 that is then inserted into the final product to form the absorbent product.

In accordance with this solution, there is therefore greater production flexibility, making it much easier to change the format and, moreover, allowing savings in overall dimensions and management by no longer having to store in stock multiple reels of airlaid material of different formats.

The recipe change in general is now simple and fast as it is enough to simply act, for example, on the amount of cellulose fibres in order to modify the sizes of the absorbent core in thickness and/or its absorption and comfort capacity, it in fact being also possible to easily add SAP even of very fine grammages.

The fact that in production the absorbent cores 201 are arranged according to a certain pitch in the tape 200 implies that, after cutting out, there is certainly a saving of wastage and noble material compared to the case in which an airlaid tape is used that is entirely suitable in itself to be able to constitute a pad. In fact, the tape 200 is not now a tape in itself suitable for the formation of the pad but, rather, the suitable part is that relative only to the absorbent cores 201 that are made spaced apart from each other according to a certain pitch.

Advantageously, therefore, each single absorbent core (201') obtained is therefore sent in line to the subsequent forming section of the final absorbent product for sanitary use in which each single said absorbent core (201') produced upstream in the same converter is inserted between at least two layers of overlapped material to form said absorbent product for sanitary use.

The obtained final absorbent product for sanitary use can be, in a non-limiting manner, for example, a pad for female use or diapers in general, for example for adults or children.

In this way, a single production line allows producing the absorbent cores, which are then, always in the same line, used to obtain the final product.

Advantageously, said step of directly producing in said converter line (1, 2, 3, 4) a continuous tape (200) comprising a plurality of absorbent cores (201) provides the operation of pouring cellulose fibres (30) inside a mould (20).

The mould, therefore, is a component belonging to the converter.

In this way, therefore, it is easily possible to change recipes as it will be possible, for example, to be able to control the amounts and quality of cellulose fibres 30 to be poured inside the mould.

In addition, thanks to this solution, it will therefore be possible to add SAP material together with cellulose fibres, thus being able to better control the degree of absorbability of the core (201) obtained.

Advantageously, the step of moving forward a first tape (22) and a second tape (23) along a processing path can be provided.

In particular, one of said first tape (22) and second tape (23) is laid on the mould (20) for receiving said cellulose fibres (30) and the other tape overlaps it for closing the content and forming the continuous tape (200) comprising said absorbent cores (201) spaced out from each other according to a certain pitch.

The forward movement of the tape can occur for example through the vacuum that retains the tapes and the finished product on a moving surface.

For example, the same mould may be in the form of a roller 20 that is rotatable about its longitudinal axis and it can be provided on its side surface for a plurality of moulds spaced according to a certain pitch.

The roller rotates in start and stop mode, thereby intermittently bringing each mould under the fall of cellulose fibres for filling it. After filling has occurred, it starts rotating again until the other mould is in position and so on. In the restart step, the fall of cellulose fibres is interrupted to resume when the mould is in position. This allows to obtain absorbent cores that are spaced according to a certain pitch, thus with the cellulose fibres used only for the formation of the absorbent cores.

This embodiment solution with absorbent cores arranged according to a certain pitch solves an important technical problem.

The traditional airlaid production lines are traditionally characterized by the fact of producing at low speed, consuming a lot of energy even due to the use of high amounts of humid air to favour the separation among the fibres and uniformity of distribution.

By their nature, therefore, these airlaid production lines are not suitable to be integrated into a "converter" line that produces the absorbent products for sanitary use but they are always external and independent lines, since the entire airlaid tape is actually a material suitable for the formation of the pad and which therefore requires, as mentioned above, a lot of energy for its production.

The solution proposed above, in which cellulose fibres are poured inside a mould intermittently to obtain a tape that has indeed absorbent cores but which are arranged according to a certain pitch, allows to save production energy and allows this solution to be integrated directly into the converter without reducing the production speeds.

In this way, simply by feeding two tapes, for example in cellulose, the final tape (200) is obtained in a versatile way which comprises, according to a certain pitch, the absorbent cores.

In order to change, for example, the size of the absorbent cores, it will be sufficient, for example, to act on the starting width of the two tapes and/or on the amount of cellulose fibres poured.

The two tapes can be independent and can be unwound from a special mother reel arranged in a special initial section (1) of the converter line as well as the two tapes could be generated from a single mother reel of tape which is then cut along its longitudinal centreline.

All this, therefore, gives enormous versatility.

Advantageously, together with the cellulose fibres (30), the step of pouring also a predetermined amount of SAP material can be provided.

SAP material, as is well known in the sector, is a highly absorbent material, in particular a polymer, so much so that SAP is precisely the acronym for *Super Absorbent Polymer.* The SAP is also known as *"water trap".* In general, SAP is a cross-linked hydrophilic polymer that can swell by absorbing a large amount of water or of aqueous solutions, up to a few hundred times its weight.

For this reason, the use of SAP is well known in the sector of the production of absorbent products for sanitary use such as indeed absorbent pads for female use as well as diapers for children and/or adults, etc.

In particular, in accordance with the invention, the SAP can be added to the cellulose fibres for thus forming a highly absorbent core (201').

The expert in the field will be able to select the best combination of cellulose fibres and SAP for modulating and therefore optimising the absorbency of the final product as a function of their needs.

For example, advantageously, in case of pouring SAP, the step of production of a first layer of cellulose fibres (30) in the mould can be provided on which the SAP is poured to form a second layer.

This second layer is then covered with a further third layer of cellulose fibres (30).

In this way, the SAP is advantageously comprised between two layers of cellulose fibres (30), thereby obtaining a highly absorbent core 201.

As introduced in the prior art, the use of very fine SAP can quite complicate the construction of a converter machine and relative process with formation of the absorbent core starting directly from cellulose fibres directly in the converter, as there may still be a risk that very fine SAP particles, for example of the order of 50 microns, can be sucked in and lost during said formation process, for example in the specific case in which the vacuum is used for the forward movement in processing.

In accordance with the solution in which the SAP layer is interposed between two layers of cellulose fibres, it also solves this problem in case of forward movement with vacuum, thus allowing a use of a particularly fine SAP, thus optimising the flexibility of the product, its comfort in use and preventing sharp tips from being able to exit from the finished product.

In all the aforementioned cases, advantageously, before coupling the first tape (22) with the second tape (23) to form said absorbent core, the step of applying a glue to at least one of said first tape and/or second tape occurs.

In this way, the coupling of the first tape with the second tape is reinforced and/or the aggregation between the fibres is reinforced. In other words, the absorbent core, which must then be cut out, or shaped, is more stable by not reopening after shaping.

Advantageously, a humidification step of the cellulose fibres can be provided before forming the absorbent core.

This further contributes to the adherence of the fibres to each other in the formed core as the humidity, subject to pressure, compacts the fibres well. This therefore ensures that after shaping the core does not open nor, even less, that the pressed core expands after pressing.

Preferably the use of glue is combined with the fibre humidification process so as to have the optimal effect of fibre adhesion and compactness but only one of the two processes could be used.

Advantageously, a pressing step of said absorbent cores (201) obtained can subsequently be provided.

In this way, advantageously, the absorbent core (201) can be compacted greatly reducing its thicknesses so as to make it suitable in the production process to constitute the absorbent part of the final absorbent product to be produced, for example a female pad.

It is in fact an important requirement that the thicknesses of the absorbent products, in particular those for female use, have very small thicknesses to be usable without discomfort.

Advantageously, said pressing step can occur before the step of cutting out said absorbent cores (201).

In this way, the continuous tape comprising said absorbent cores arranged according to a certain pitch can easily enter the section (3) that acts on each core by pressing it to the set value.

The upstream use of glue and/or humidification of the fibres greatly facilitates the achievement of thin thicknesses during the pressing step, maintaining the core at its reduced thickness after pressing and subsequent shaping.

As indicated above, the method in accordance with the invention is extremely versatile and therefore it is possible, for example, to provide for a pouring of an amount of cellulose fibres (30) to form said absorbent cores 201, which amount can be comprised between 50 grams per square metre and 300 grams per square metre, extremes included.

In fact, the Applicant has surprisingly found that these values are optimal for obtaining a core with excellent absorbency and in any case compressible to very thin thickness values.

In particular, the thickness of the absorption core (30) in accordance with the above can in fact be comprised, after the pressing operation, between 0.5mm and 3mm extremes included.

Advantageously, in accordance with the above, SAP, which, for example, can have particle sizes from 50 microns to 400 microns extremes included, can also be added to the cellulose fibres 30.

Such a fine particle size makes it possible to obtain thin, very comfortable and highly absorbent products.

In particular, precisely to increase the comfort of use in the final product, it is preferable that with such thin particle sizes the SAP layer is interposed, as already indicated above, between two layers of cellulose fibres, thus remaining trapped and thus avoiding the formation of tips exiting from the final product that can sting.

In accordance, therefore, with a possible preferred configuration, cellulose fibres can be used in an amount comprised between 50 grams per square metre and 300 grams per square metre with the addition of an SAP whose particle size is preferably comprised between 50 microns and 400 microns.

In addition, advantageously, this SAP can be arranged between two layers of cellulose fibres.

This therefore allows to obtain a comfortable absorbent core with high absorbency avoiding that the thin SAP is sucked in case of forward movement of the tape 200 by means of vacuum.

Advantageously, the method described makes it possible to obtain a wastage size (i.e. the part of waste material after cutting out the absorbent core) lower than 6mm, preferably lower than or equal to 5mm.

This is because the production in the converter directly of the product allows to adjust already at the start, as a function of the recipe to be obtained, the suitable size of the absorbent core depending on the expected mould and the use of suitable starting tapes. The mould also follows the typical shape of the pad (201') to be inserted into the finished product. It follows that the single core obtained has a shape and size very close to the pad to be inserted into the final absorbent. In this way, in the operation of cutting out the absorbent core 201 from the tape 200, the wastage is really minimal compared to that of the prior art.

There is therefore advantageously a considerable reduction in the waste material that generally results in a noble material. This therefore implies cost savings.

Advantageously, the cutting out operation of the absorbent cores provides the operation of cutting out each absorbent core (201) inside its perimeter.

In this way, advantageously, the single absorbent core does not have a perimeter edge without cellulose fibres.

Therefore, an object of the present invention is also a production line (1, 2, 3, 4) for manufacturing an absorbent product for sanitary use.

In accordance with the invention, said production line comprises in succession to each other at least the following sections:

A section (2) configured for the production of a continuous tape (200) comprising a plurality of absorbent cores (201) arranged between them according to a certain pitch;

A cutting section (4) configured to separate from the continuous tape (200) said absorbent cores (201), thus obtaining single absorbent cores (201');

A forming section configured to obtain the final absorbent product for sanitary use.

In this way, all the technical effects already discussed above are obtained.

In particular, as already mentioned, the absorbent core is now produced directly in line in the converter.

In this way, it is no longer necessary to have to use airlaid mother reels already made upstream and stored in the warehouse, since the same converter line can now produce said absorbent core 201 that is then inserted into the final product to form the absorbent product.

In accordance with this solution, there is therefore greater production flexibility, making it much easier to change the format and, moreover, allowing savings in overall dimensions and management by no longer having to store in stock multiple reels of airlaid material of different formats.

The recipe change in general is now simple and fast as it is enough to simply act, for example, on the amount of cellulose fibres in order to modify the sizes of the absorbent core in thickness and/or its absorption and comfort capacity, it in fact being also possible to easily add SAP even of very fine grammages.

Advantageously, therefore, the forming section configured to obtain the final absorbent product for sanitary use is therefore configured to be able to insert between at least two layers of material said single absorbent core (201') to thus form said final absorbent product for sanitary use, for example a pad for female use or diapers in general for example for adults or children.

Advantageously, said section (2) configured for the production of the continuous tape (200) can comprise:

At least one mould (20) adapted to receive at least cellulose fibres (30) and/or any SAP material;

Means (10, 21) adapted to pour inside the at least one mould (20) said at least one cellulose fibre (30) and/or any SAP material.

In this way it is possible to pour the cellulose fibres and/or SAP according to a specific recipe into the mould to obtain an absorbent core.

Advantageously, at least one first path (24, 25) can be further comprised which is adapted to allow feeding of at least one first tape (22) towards said at least one mould (20) in such a way that said at least one first tape (22) can be laid in use on said mould for receiving said at least cellulose fibres (30) and/or any SAP material;

In the same way, advantageously, at least one second path (24, 25) can be provided which is adapted to allow feeding of at least one second tape (23) in such a way that said at least one second tape (23) can overlap in use said first tape after pouring said at least cellulose fibres (30) and/or any SAP material on the first tape thus forming said continuous tape (200) comprising said absorbent cores (201).

In this way, in a versatile way, the production of the continuous tape comprising the absorbent cores (201) is obtained directly in the converter 100, or said production line 100, without, therefore, the need to have to provide such airlaid reels upstream of the machine.

In particular, the converter itself provides, according to a recipe that can be modified based on needs, to make absorbent cores arranged according to a certain pitch.

Advantageously, at the exit of said section (2) for producing a continuous tape (200) comprising a plurality of absorbent cores (201), it can be provided, in line, for a subsequent pressing section (3) configured to press said absorbent cores (201) in such a way as to reduce the thickness thereof.

In this way, a comfortable final product can be created, since the thickness value of the absorbent core can be controlled.

Advantageously, a humidifying device (10') can be provided which is arranged in such a way, in use, as to humidify the cellulose fibres (30) before forming the absorbent core.

This favours the adhesion of the fibres when they are compressed to form the absorbent core, favouring the achievement of thin thicknesses in compression and avoiding flaking of the obtained core once shaped.

Advantageously a glue distributing device configured to distribute an adhesive material may be provided.

Advantageously, it can be arranged in such a way as to be able, in use, to distribute said adhesive material on at least one of said first tape and second tape during their forward movement along respectively the first path (24, 25) and the second path (24, 25) before forming the absorbent core.

In this way, the adhesion of the tapes to the fibres is optimised and this favours the achievement of thin thicknesses in compression and avoids flaking of the obtained core once cut.

The use of glue and/or humidification of the fibres greatly facilitates the achievement of thin thicknesses during the pressing step, maintaining the core at its reduced thickness after pressing and subsequent shaping.

Although therefore the combined use is preferable, either only the humidifying device (10') or only the distribution of glue can still be activated while still having a benefit.

This mechanism, as mentioned, favours the pressing operation by contributing to the achievement of thin thickness values.

Advantageously, subsequent to said pressing section (3), said cutting section (4) can be provided in line.

In this way, the absorbent cores are first easily transported to the pressing section as they are connected to a single tape 200 and then, once the pressing work is completed, they are cut out, that is, separated from the mother tape to which they are connected.

Advantageously, said cutting section (3) can be configured to remove a material wastage from said tape (200) that is lower than or equal to 6mm of material, preferably lower than or equal to 5mm of material.

In this way, the waste of noble material is reduced compared to prior art technology that uses starting airlaid reels.

In particular, the use of a mould of suitable shape with formation of the core directly in the mould allows to obtain a single product (201') of precise size such that its cutting out leads to a minimum waste with respect to solutions of prior art in which the entire starting airlaid tape is suitable material to constitute the core but which is then cut to size with a high product waste.

Advantageously, subsequent to said cutting section (4), a section for inserting the cores (201) between at least two layers of material for the formation of the final absorbent product for sanitary use is provided in line.

In this way, the same converter, that is, the same line, produces the finished product without the use of starting airlaid reels but rather by producing the cores itself with great flexibility so that recipe and/or sizes as well as other possible parameters thereof can be controlled.

Advantageously the mould (20) can be in the form of a rotatable roller.

In this way the roller rotates in steps (i.e. start and stop) allowing the cellulose fibres to be poured into each mould obtained around the circumference of the roller.

The circumference of the roller can in fact provide for a plurality of moulds.

The mould follows the shape of the final core to be inserted in the final product with a size close to the core that actually has to be inserted, therefore having a minimum waste.

In this way, a single continuous tape 200 is obtained in which the absorbent cores 201 are present and arranged according to a certain pitch, i.e. spaced apart from each other according to a certain pitch.

The forward movement of the obtained tape can occur through, for example, the aid of the vacuum acting through holes present in the roller 20 whereby keeping the obtained tape 200 adherent and moving it forward thanks to the rotation of the roller 20.

Other forward movement systems, known in the sector, would be possible.

Advantageously, the pressing section (3) can comprise at least one pair of counter-rotating rollers (31) arranged in front of each other at a certain distance.

In this way the distance gap between the two rollers can be easily adjusted so as to adjust the thickness of the absorbent core.

Advantageously, the mould (N, 20) can comprise a rotatable roller (20) on the side surface of which one or a plurality of niches (N) are obtained.

Advantageously, the roller is arranged under a duct (21) that conveys the cellulose fibres 30 and/or SAP from a distributor / shredder device (10).

Advantageously, the rotation of the roller (20) is controlled in such a way as to be of start and stop type.

According to an advantageous aspect of the invention, an object of the invention is also a tape (200) consisting of at least a first tape (22) and a second tape (23) overlapped on each other to form said tape (200), which tape (200) comprises a plurality of absorbent cores (201) arranged according to a certain pitch from each other and of which each absorbent core contains a certain amount at least of cellulose fibres (30) or a combination of cellulose fibres and SAP.

According to an advantageous aspect of the invention, an object of the invention is also the use of a tape (200) for manufacturing absorbent products for sanitary use, wherein said tape (200) consists of at least a first tape (22) and a second tape (23) overlapped on each other to form said tape (200), which tape (200) comprises a plurality of absorbent cores (201) arranged according to a certain pitch from each other and of which each absorbent core containing a certain amount at least of cellulose fibres (30) or a combination of cellulose fibres and SAP and wherein said use provides for the separation of said absorbent cores from the tape in such a way as to obtain single absorbent cores (201') that are used as a pad of the final absorbent product for sanitary use.

### Brief description of the drawings

Further features and advantages of the present method and relative production line (converter) according to the invention, will become clearer with the following description of some of its embodiments, made by way of non-limiting example, with reference to the accompanying drawings, in which:
- Figure 1 shows a schematization of a line in accordance with the invention highlighting an initial section 1, a subsequent section 2 used to form the absorbent core and a subsequent pressing section 3;
- Figure 1A shows a roller 20, present in the section 2, which forms the mould for obtaining the absorbent core, and into which the fibres 30 are poured; the roller has around its side surface a plurality of niches (N) or seats, each forming a mould;
- Figure 2 shows a further schematization of converter in accordance with the invention showing both the aforementioned section 2 for the formation of the absorbent cores arranged spaced apart from each other according to a certain pitch, followed by a pressing section 3 (or pressing section as the case may be) and a subsequent cutting out or cutting section 4 as the case may be;
- Figure 3 and Figure 4 schematizes the "absorbent core" product obtained in the mould in accordance with the invention, i.e. the formation of a continuous tape comprising the absorbent cores 201 arranged according to a certain pitch;
- Figure 5 schematizes the cutting out or cutting section that through a blade 221 separates each absorbent core 201 from the tape 200 with a minimum wastage; the dotted line shows the cut made on each absorbent core to obtain the single absorbent core (201') separated from the tape.

### Description of some preferred embodiments

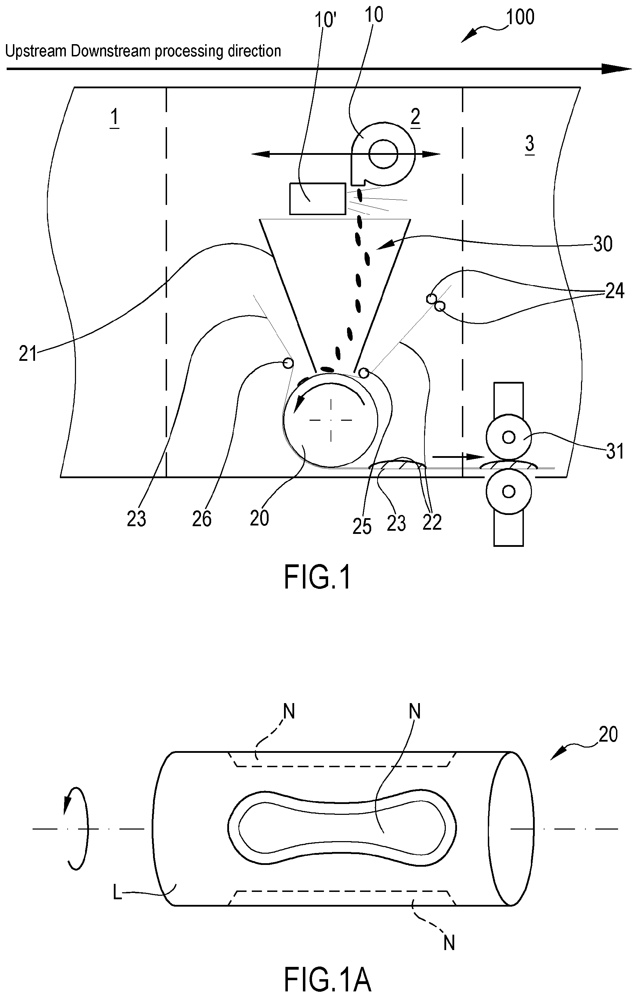

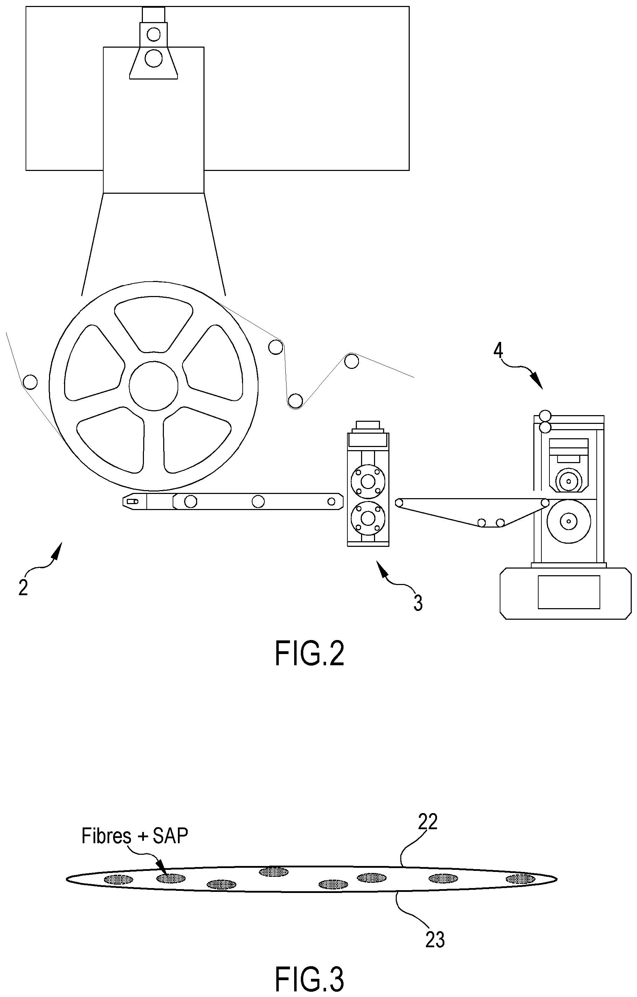

Figure 1 and Figure 2 both schematize a solution in accordance with the invention.

Figure 1, with the numbering 100, indicates a portion of the production line (i.e., the converter) for the production of absorbent products for sanitary use.

Sections are therefore highlighted in succession (1, 2, 3) some of which are already known in the field in order to obtain the finished product and therefore not described in detail here.

The processing direction is therefore from "Upstream" (i.e. origin of the converter line) towards "Downstream" therefore towards the outlet section of the finished product.

The section 1 can be, as clarified below, a section in which the starting tape(s) is/are unrolled for the in-line formation of a pad 201, i.e. a single absorbent core 201' which is then inserted precisely to form the pad of the final absorbent product.

For this purpose, the section 1 (not shown in the figure in detail) can provide standard unwinders which, however, now, in accordance with the invention, do not directly unwind a ready airlaid tape but they unwind simple starting tapes (for example in TNT or cellulose) for the formation of an "ad-hoc" core as a function of needs.

The sections 2, 3 and 4 follow which are therefore described in detail below for the formation of the absorbent core produced directly in the converter line according to the specific recipe and then the line continuing with the use of the core, as per prior art, for the formation of the finished product.

Therefore, entering more into the descriptive detail, and as thus schematized in Figure 1, there is now, in accordance with the invention and on board the converter machine 100, a specific section (indicated by way of non-limiting example with the numbering 2) which contains in its inside a mould 20 for the in-line formation of the absorbent cores 201, i.e. cellulose fibres with any SAP.

The mould is therefore integrated inside the converter line 100 and therefore, in this case and in accordance with the invention, it is no longer necessary to use airlaid mother reels constituting the pad once cut, since this is prepared "on site" in a special section 2 as described herein.

So, actually, according to the new production cycle, the absorbent core is produced on site within the converter which then continues within the converter 100 to be used to form the final absorbent product according to a final part of the cycle already known.

What is mentioned above, and as will be clarified immediately below, gives great flexibility to the production line 100, allowing the formats to be easily modified, also with considerable material savings.

The mould 20, as shown in Figure 1, can be for example in the form of a rotatable wheel (i.e. a roller) 20 to which two starting tapes (22, 23) of suitable material converge, for example made of a paper nature such as preferably cellulose or the like or in materials such as non-woven fabric.

Figure 1 therefore schematizes in the section 2 a sort of funnel or channel 21 that serves to convey the material, i.e. the cellulose fibres 30 that will form, in the production process, the absorbent cores.

The material that is poured into the mould 20 is therefore in the form of cellulose fibres 30.

As schematized in Figure 1, the fibres 30 can be poured into the mould with any distributor/shredder 10 that can shred the cellulose to form the fibres and pour the shredded product, for example by gravity or, better, by ejecting it through a pressure at the exit of a nozzle. Figure 1 therefore schematizes a distributor/shredder device 10 which pours cellulose fibres 30 into the mould 20 through a nozzle.

The funnel or duct 21 typically helps precisely convey the fibres 30 on the mould.

The distributor / shredder device can also integrate a humidifying device (10') capable of sending a humidified flow to the cellulose material to humidify it.

The humidification operation can occur before the cellulose is shredded into fibres or as soon as it is shredded but preferably before it reaches the mould. The humidifying device (10') can therefore be integrated into the distributor / shredder 10 or it can be arranged separately, for example in such a way as to intercept the cellulose fibres at the exit of the distributor / shredder device.

This allows, in the subsequent pressing step, to compact the whole much better thanks to the presence of humidity in the cellulose fibres.

The mould is preferably obtained in a rotatable wheel 20 and more precisely a cylindrically shaped roller provided with a side surface (L). On its side surface, niches (N) can be obtained as schematized in Figure 1A.

These niches (N) form the collection site for the fibres 30 that fall by gravity, as schematized in Figure 1 or Figure 2, thus forming each niche in a mould.

The mould has a geometry such that it replicates the shape and size of the final pad as much as possible. The shape of Figure 1A shows in fact a niche N with the typical shape of the pads of the absorbent products.

When the roller 20 rotates, for example in steps, it can stop each niche (N) under the duct 21 in order to receive the fibres 30.

More in particular, a first tape 22 fed by the section 1 and moving along a feeding path (for example obtained by means of guide rollers) is laid on the niche N as schematized in Figure 1 in such a way that it covers the niche that has the function of a mould, being filled with falling fibres 30.

The tape is laid perfectly in the niche, thus replicating its shape, for example because it is sucked in by a suction.

A second tape 23 is fed towards the rotatable mould 20 by a second feeding path with respect to that of the first tape, in such a way as to overlap the first tape 22 that covers the niche containing the fibres 30. An absorbent core 201 is thus obtained as better described below.

Said tapes 22 and 23 overlapped in adherence to the roller 20 move integrally with the roller 20. For example, this is because the roller is provided with suction holes to keep the tape adhering to it, so rotating the roller causes it to move forward towards the following sections. In this way, the two overlapped tapes at the niche and containing the fibres move towards the further processing sections.

The roller 20 is, as mentioned, provided with a plurality of niches N such that cyclically, during its rotation, it carries under the funnel 21 a respective niche with the tape laid on it to receive the fibres and then the second tape is applied on top of the first to close the fibres.

In other words, the second tape 23 overlaps the first tape determining in output a product made by the two tapes 22 and 23 overlapped on each other and containing in their inside the fibres in the zone of the tape that was in the niche. The roller rotates in start and stop mode and when the roller rotates to transfer a next niche under the duct 21, in this step the pouring of cellulose fibres is interrupted. In this way a tape is obtained having absorbent cores 201 spaced out from each other according to a certain pitch.

The whole then travels towards a section 3 where a pressing element 31, for example a vertically movable cylinder, crushes the obtained core, which is thus pressed to reach thicknesses as thin as possible.

In order to optimize the core obtained, the two tapes 22 and 23, before being coupled of course, can pass in contact with a glue distributor roller in such a way as to adhere to each other in the best way for the formation of the core of Figure 3.

Then a step follows of cutting to size always in line before sending the single core (201') obtained to form the final absorbent product.

Figure 1A therefore shows a possible schematization of the roller conforming the mould, that is, of the roller 20 that has the niches N on its side surface, each one constituting a mould.

Figure 2 better schematizes the section 2 described where the formation of the product consisting of the two overlapped tapes filled with fibres occurs, followed by the pressing section 3 and the section of cutting 4 to size.

The subsequent sections are not indicated as they are part of the prior art in the process of obtaining the absorbent product for sanitary use, as in any case will be mentioned in the final part of this description.

Figure 3 schematizes the finished product at the exit of each mould niche N, which product can for example provide two layers of cellulose consisting of the two starting tapes 22 and 23 within which also SAP granulate can optionally be trapped, in addition to the fibres 30.

In particular, the process may provide for pouring cellulose fibres into the mould to create a bed of fibres on which the SAP can then be poured and then proceeding with pouring other fibres over the SAP to make the second layer.

In this way, the SAP is enclosed between two layers of fibres, remaining trapped and this allows the use of SAP with very low particle size because, being trapped, it is not sucked in and does not come out, risking the creation of sharp tips.

In all of the above cases, the fibres may be humidified and/or at least one of the two starting tapes may be provided with a layer of adhesive.

Obviously, as already mentioned, the mould is preferably of the start and stop type, i.e. it stops under the duct or funnel 21 to be filled and the filling can provide for the fall of fibres and/or SAP, for example according to the above, i.e. a first layer of fibres on which the SAP is poured and then followed by a further layer of fibres for covering.

As already mentioned above, in order to press the cellulose fibres 30 giving compactness and thinness to the product being formed inside the mould, it is possible, for example, to exploit the well-known *"hydrogen bonding"* system, i.e. adhesion between the fibres given by a chemical bond with hydrogen bridge that is generated between the fibres in particular by subjecting the fibres to high pressures and the presence of humidity.

In this sense, humidifying through the device 10' creates a hydrogen bonding effect.

The term *Hydrogen bonding* therefore means the hydrogen bridge that is created under pressure with the presence of humidity.

Figure 1 therefore schematizes a pressing system with the numbering 31.

Such a pressing system may be in the form of a translatable piston which crushes the tape. However, in a preferred solution and as shown in the figures, such a system can simply provide at least one pair of rollers 31 opposed to each other and counter-rotating. They are therefore arranged in front of each other at a certain adjustable distance from each other (a gap). The distance between them can be of the order of a few tenths of millimetres as well as something close to zero. The two counter-rotating rollers, by rotating, take at the inlet the tape and crush it between them, thus reducing the thickness of the absorbent cores (201).

The use of humidity and adhesive favours maintaining the compressed thickness after the completion of this operation and after cutting out.

The Applicant has experimentally verified, and obtained excellent results, through the use of cellulose fibres 30 whose grammage is comprised in a range between 60 grams per square metre and 500 grams per square metre, preferably between 50 grams per square metre (gsm) and 300 grams per square metre (gsm), extremes included.

Obviously, the quantity poured is smaller and the thickness achievable for said product is smaller.

In particular, the Applicant has surprisingly found that, in accordance with the present method and using the amounts indicated above, it is possible to obtain thicknesses of the single absorbent core 201', comprised between 4 mm and 0.2 mm, preferably between 3 mm and 0.5 mm extremes included, hence very thin thicknesses.

This is obtainable by dosing the pressure value in the crushing and remaining in the above ranges of fibres, preferably between 50 grams per square metre and 300 grams per square metre.

Therefore, if the starting thickness of the product, before pressing, is 10 mm, then it is possible to press it, for example, up to 3 mm or 4 mm, assuming, for example, a filling value of 300 grams per square metre.

If the starting thickness is 3mm, for example with a minimum filling value of 50 grams per square metre, then the thickness can also reach for example 0.5 mm or said 0.2mm.

Obviously, based on how much SAP is added, these thicknesses may be modified.

Any SAP present can be in percentages from 0% to 70% with respect to the total weight of the absorbent core.

The particle size of the SAP can be from 50 microns to 800 or 900 microns extremes included but preferably below 400 microns as the fine particle size makes the presence of SAP less evident.

In this case, as mentioned, a layered distribution of SAP comprised between cellulose layers may be preferable.

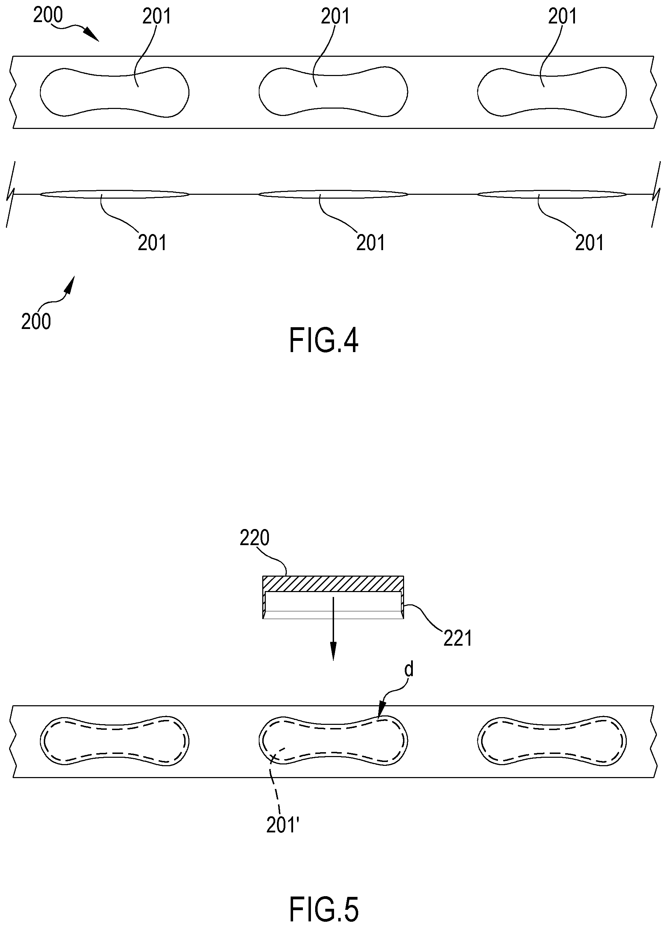

The finished product, always obtained in line directly in the converter machine 100, therefore appears as a continuous tape 200 of product having the portions filled as described and constituting the actual absorbent core 201 which will then form the final absorbent product, as schematically shown in Figure 4.

In particular, Figure 4 shows a product tape 200 having precisely the absorbent cores 201 obtained through the mould 20 and therefore spaced out from each other at a mutual distance according to a certain pitch along the product tape, a product tape that we remind to be obtained by overlapping the two simple starting tapes 22 and 23.

It is evident that this solution is highly flexible since already in line, within the converter, a plurality of cores are obtained whose fibre dosage, SAP quantity, size and geometric shape can be controlled upstream during production simply by selecting the starting tapes 23 and 22, the mould conformation as well as the quantities of fibres and/or SAP to be poured. Furthermore, the method allows to create absorbent cores according to a certain pitch such that, when they are separated from the tape, the waste is minimal compared to the case of prior art in which the entire starting tape is an airlaid that can potentially entirely constitute the pad of the final product.

Figure 4 shows, therefore, by way of example, the product tape 200 (seen from above and from the side) where it is evident how, according to a certain pitch, the actual absorbent cores 201 are distributed which then actually represent the absorbent part of the finished product (i.e. the pad).

In order for this product, which is de facto the one at the exit of the section 3 of Figure 2, to be able to continue its processing path in the converter in order to form the absorbent core of the final absorbent product, i.e. the female pad or the diaper, it must be separated from the tape by cutting it to size with a suitable geometry.

For this purpose, the tape 200 enters the subsequent section 4 where it is cut or cut out as the case may be, for example by means of a special cutter similar to a die, cutting the excess tape thereof out.

Figure 5 schematizes, in this regard, a section 4 in which a die 220 with blade 221 of suitable geometry is used to cut out the absorbent core 201.

In fact, the figure shows the tape and with a dotted line it shows the cut that cuts out the core separating it from the tape.

The cut occurs inside the perimeter of the absorbent core (201) following the shape of the perimeter, as the single core (201') must necessarily comprise cellulose fibres since it will then be inserted into the final product.

Therefore, if the cut occurred outside the perimeter of the core (201), there would be a section (d) without cellulose fibres and consisting only of the two simple overlapping starting tapes (22, 23).

It is then proceeded with cutting out (therefore cutting) an internal zone with a blade that follows the shape of the core (201), therefore of suitable geometry and size. This allows to have a truly minimal waste (d), indicated in Figure 5, considering that the starting core (201) made is very close to the final size of the single and separate core (201').

In this way it is possible to manage a much more precise cut, thus obtaining a considerable saving of waste noble material compared to the prior art in which the starting tape is not shaped and it is an airlaid tape that in fact constitutes a potential absorbent core in its entirety. It follows that cutting the airlaid according to prior art results in a loss of a lot of wastage.

More particularly, the cutting blade can intercept the product extracted from the mould by cutting it out according to the exemplary form of Figure 5.

The wastage (that is, the waste) can easily be lower than 5mm and even below this value, therefore much lower than wastages that use a continuous tape of airlaid already ready upstream and inserted in the form of a mother reel.

The production process described, in fact, allows to select the best recipe, i.e. width of the starting tapes 22 and 23 as well as type and quantity of SAP and fibres 30, conformation and mould size thereby also allowing to make tapes 200 (for example a single continuous tape 200 as well as several continuous tapes 200 for example placed in parallel) whose wastage is really minimal.

Figure 5 shows, in fact, the measure "d" of waste or wastage that can be lower than 5mm or even lower than this value.

The forward movement of the tape from the formation of the absorbent core (201) up to its cutting can occur for example by means of counter-rotating rollers and/or suction and in any case according to the means known in the sector for the forward movement of tapes in general.

The process continues in the subsequent sections of the converter not depicted here in the figure for the sake of simplicity and which are known per se.

In particular, each single absorbent core (201'), once separated from the others, is fed towards the formation section of the final absorbent product, for example the pad for female use.

It is inserted between two layers of material which then deliver the finished product, for example the female pad.

The whole proceeds towards a packaging section.

## Claims

1. A method for manufacturing, in a production line (100), an absorbent product for sanitary use, the method comprising the steps of:
- Directly producing in said production line a continuous tape (200) comprising a plurality of absorbent cores (201) arranged in the tape spaced apart from each other according to a certain pitch;
- Cutting out from said tape (200) the absorbent cores (201) in such a way as to separate them from the tape (200) and obtain single absorbent cores (201');
- Sending said single absorbent cores (201') towards a subsequent manufacturing section of the final absorbent product for sanitary use.

2. The method according to claim 1, wherein said single absorbent core (201') is inserted between at least two layers of material to form said final absorbent product for sanitary use.

3. The method according to claim 1 or 2, wherein said step of directly producing in said production line (100) a continuous tape (200) comprising a plurality of absorbent cores (201) provides the operation of pouring cellulose fibres (30) inside a mould (N, 20).

4. The method according to claim 3, wherein the step of moving forward a first tape (22) and a second tape (23) along a processing path is provided and of which one of said first tape (22) and second tape (23) is laid on the mould (N, 20) for receiving said cellulose fibres (30) and the other tape overlaps it for closing the content and forming a continuous tape (200) comprising said absorbent cores (201) spaced out from each other according to a certain pitch.

5. The method according to claim 3 or 4, wherein, together with the cellulose fibres (30), the step of pouring also a predetermined amount of SAP material is provided.

6. The method according to claim 5, wherein, in case of pouring SAP, the step of production of a first layer of cellulose fibres (30) is provided on which the SAP is poured to form a second layer, which second layer is then covered with a further third layer of cellulose fibres (30).

7. The method, according to one or more of the previous claims from 4 to 6, wherein before coupling the first tape (22) with the second tape (23) to form said absorbent core (201) the step of applying a glue to at least one of said first tape (22) and/or second tape (23) occurs for at least reinforcing coupling of the first tape (22) with the second tape (23).

8. The method according to one or more of the previous claims, wherein a humidification step of the cellulose fibres (30) is provided before forming the absorbent core (201).

9. The method, according to one or more of the previous claims, wherein a pressing step of said obtained absorbent cores (201) is provided, preferably through the passage of the tape (200) into the gap formed by at least one pair of counter-rotating rollers (31) arranged in front of each other.

10. The method according to claim 9, wherein said pressing step occurs before the step of cutting out said absorbent cores (201).

11. The method according to one or more of the previous claims, which provides pouring an amount of cellulose fibres (30) into said mould (20) comprised between 50 grams per square metre and 300 grams per square metre.

12. The method according to one or more of the previous claims, wherein the thickness of the absorption core (30) is comprised, after the pressing operation, between 0.5mm and 3mm extremes included.

13. The method according to one or more of the previous claims, wherein the wastage size after cutting out operation is lower than 6mm, preferably lower than or equal to 5mm.

14. The method according to one or more of the previous claims, wherein the cutting out operation of the absorbent cores provides the operation of cutting out each absorbent core (201) inside its perimeter.

15. A production line (100) for manufacturing an absorbent product for sanitary use, said production line (100) comprising in succession to each other at least the following sections:
- A section (2) configured to produce a continuous tape (200) comprising a plurality of absorbent cores (201) arranged between them according to a certain pitch;
- A cutting section (4) configured to separate from the continuous tape (200) said absorbent cores (201), thus obtaining single absorbent cores (201');
- A forming section configured to obtain the final absorbent product for sanitary use.

16. The production line (100) according to claim 15, wherein said forming section configured to obtain the final absorbent product for sanitary use is configured to insert between at least two layers of material said single absorbent core (201') to form said final absorbent product for sanitary use.

17. The production line (100) according to claim 15 or 16, wherein said section (2) configured to produce a continuous tape (200) comprises:
- At least one mould (N, 20) adapted to receive at least cellulose fibres (30) and/or any SAP material;
- Means (10, 21) adapted to pour inside said at least one mould (N, 20) said at least cellulose fibres (30) and/or any SAP material;
- At least one first path (24, 25) adapted to allow feeding of at least one first tape (22) towards said at least one mould (N, 20) in such a way that said at least one first tape (22) can be laid in use on said mould (N, 20) for receiving said at least cellulose fibres (30) and/or any SAP material;
- At least one second path (24, 25) adapted to allow feeding of at least one second tape (23) in such a way that said at least one second tape (23) can overlap in use said first tape after pouring said at least cellulose fibres (30) and/or any SAP material on the first tape thus forming said continuous tape (200) comprising said absorbent cores (201) arranged according to a certain pitch.

18. The production line (100), according to one or more of the previous claims from 15 to 17, wherein at the exit of said section (2) it is provided, in line, for a subsequent pressing section (3) configured to press said absorbent cores (201) in such a way as to reduce their thickness.

19. The production line (100), according to one or more of the previous claims from 15 to 18, wherein a humidifying device (10') is provided arranged in such a way, in use, as to humidify the cellulose fibres (30) before forming the absorbent core.

20. The production line (100), according to one or more of the previous claims from 15 to 19, wherein a glue distributor device is provided which is configured to distribute an adhesive material and arranged in such a way as to be able, in use, to distribute said adhesive material on at least one of said first tape and second tape during their forward movement along respectively the first path (24, 25) and the second path (24, 25) before forming the absorbent core.

21. The production line (100), according to one or more of the previous claims from 15 to 20, wherein, subsequent to said pressing section (3), said cutting section is provided in line..

22. The production line (100), according to one or more of the previous claims from 15 to 21, wherein said cutting section (3) is configured to separate the absorbent core (201) from the tape (200) and remove a material wastage from the absorbent core (201) that is lower than or equal to 6mm of material, preferably lower than or equal to 5mm of material.

23. The production line (100), according to one or more of the previous claims from 15 to 22, wherein said forming section configured to obtain the final absorbent product for sanitary use is provided subsequent to said cutting section (4).

24. The production line (100), according to one or more of the previous claims, wherein the pressing section (3) comprises at least one pair of counter-rotating rollers (31) arranged in front of each other at a certain distance.

25. The production line, according to one or more of the previous claims, wherein the mould (N, 20) comprises a rotatable roller (20) on the side surface of which one or a plurality of niches (N) are obtained, the roller being arranged under a duct (21) that conveys cellulose fibres and/or SAP from a distributor / shredder device (10), the rotation of the roller (20) being controlled in such a way as to be of start and stop type.
